# EUROPEAN PATENT APPLICATION

(11) **EP 1 375 465 A1**
(43) Date of publication of application: **02.01.2004**
(21) Application number: 02708747.7
(22) Date of filing: 01.04.2002
(51) Int. Cl.: C07C 49/413, C07C 49/503, C07C 49/517, C07C 49/717, C07C 49/747, C07C 49/753, C07C 49/84, C07C 69/76, C07C 205/60, C07D 213/16, A61K 31/12, A61K 31/235, A61K 31/24, A61K 31/4402, A61P 1/04, A61P 1/16, A61P 3/10, A61P 13/12, A61P 17/00

(54) **CYCLOOCTANONE DERIVATIVE AND CYCLODECANONE DERIVATIVE, AND USE THEREOF**

(30) Priority: 03.04.2001 JP 2001104075
(71) Applicant: Eisai Co., Ltd., Tokyo 112-0002 (JP)
(72) Inventor: EGUCHI, Yoshihito, Kashiwa-shi, Chiba 277-0863 (JP); CHIBA, Ken-ichi, Tsuchiura-shi, Ibaraki 300-0038 (JP); GOTO, Masaki, Tsuchiura-shi, Ibaraki 300-0841 (JP); OBAISHI, Hiroshi, Tsukuba-shi, Ibaraki 305-0035 (JP); KUBOI, Yoshikazu, Tsukuba-shi, Ibaraki 305-0035 (JP)
(74) Representative: HOFFMANN - EITLE
(86) International application number: PCT/JP2002/003258
(87) International publication number: WO 2002/081420

(57) **Abstract**

A pyrrolidine derivative, which is an intermediate for a quinuclidine derivate side chain useful as a squalene synthase inhibitor, and producing method thereof are disclosed. A pyrrolidine derivative (X) represented by the following formula (X) (wherein R¹ and R² each represents a hydroxy group, C₁₋₆ alkoxy groups or the like; R³ represents a hydrogen atom, a benzyl group or the like); or a salt thereof or a hydrate thereof:

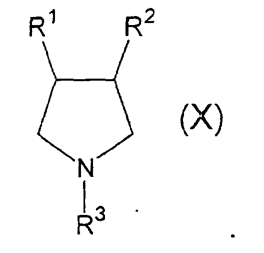

## Description

### Technical Field

The present invention relates to pyrrolidine derivatives, which are synthesis intermediates for quinuclidine derivatives useful as squalene synthetase inhibitors, for prevention and treatment of hyperlipemia such as arteriosclerotic disease, ischemic heart disease and the like, and a process for producing the pyrrolidine derivatives.

### Background Art

At present, an HMG-CoA reductase inhibitor is widely used as a cholesterol-decreasing agent. Since this inhibitor produces an effect on metabolic materials other than cholesterol, a side effect thereof is becoming a problem. A squalene synthetase functions in the lower course than an HMG-CoA reductase and an isoprene synthesis system in the biosynthesis system of cholesterol. Therefore, it is expected that the squalene synthetase overcomes the side effect problem of the HMG-CoA reductase inhibitor. However, no squalene synthetase inhibitor which produces only a small side effect and exhibits sufficient efficacy as hyperlipemia therapeutic agent has been created before.

After eager researches, the present inventors found that specific squalene synthetase inhibitors are useful as a hyperlipemia therapeutic agent, and then filed a Patent Application about the squalene synthetase inhibitors (WO 01/23383). Among squalene synthetase inhibitors, quinuclidine derivatives comprising pyrrolidine derivatives have a superior squalene synthetase inhibiting effect and are specifically useful.

In a producing process described in the Patent Application (WO 01/23383), a pyrrolidine derivative of the present invention including a compound of the present invention, 3-hydroxy-4-methoxypyrrolidine or the like, which is concerned with the present invention, is not used as an intermediate. In the process, a step of conversion of a substituent in a pyrrolidine ring is used after obtaining the intermediate. Therefore, this process is unsatisfactory as an industrial process for producing the quinuclidine derivative in light of a large number of the steps included in the process and the yield thereof as a whole. Pyrrolidine derivatives (X) including 3-hydroxy-4-methoxypyrrolidine is new as a racemic body or an optically active substance, and has not been known so far.

### Disclosure of the Invention

An object of the present invention is to provide a useful intermediate and a useful producing process therefor in the process for producing the above-mentioned quinuclidine derivative.

Further, an object of the present invention is to provide 3-hydroxy-4-methoxypyrrolidine and the producing process therefor, which is useful for industrial producing process of the above-mentioned quinuclidine derivative (WO 01/23383), and which shortened the producing steps of the above-mentioned quinuclidine derivative (WO 01/23383).

More, an object of the present invention is to provide intermediates, 3-hydroxy-4-methoxypyrrolidine and N-benzyl-3-hydroxy-4-methoxypyrrolidine useful in the process for producing the above-mentioned quinuclidine derivative, and the producing process of the intermediates.

Taking the above-mentioned things into consideration, the inventors have found out the following useful intermediates and producing process therefor in the process for producing the above-mentioned quinuclidine derivative after eager researches. The present invention relates to the following inventions <1> to <16>:
<1> A compound represented by the following formula (X). 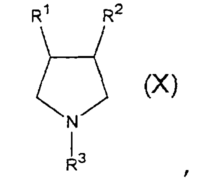 [wherein each of R¹ and R² independently means a halogen atom, a hydroxyl group or a group represented by formula -O-R¹⁰ (wherein R¹⁰ means C₁₋₆ alkyl groups which may have 1 to 3 substituents selected from a substituent group A consisting of a halogen atom, a hydroxyl group, a methoxy group, a phenyl group, C₃₋₈ cycloalkyl groups and C₁₋₆ alkoxy groups, or C₃₋₈ cycloalkyl groups which may have 1 to 3 substituents selected from the substituent group A);
   R³ means a benzyl group which may have 1 to 3 methoxy groups or nitro group(s) as substituent(s), or a hydrogen atom;
   with the proviso that a case (1) where R¹ and R² are the same with each other, and a case (2) where one of R¹ and R² is a hydroxyl group and another is an ethoxy group or a chlorine atom are excluded); or a salt thereof or a hydrate thereof.
<2> A compound represented by the following general formula (X1); 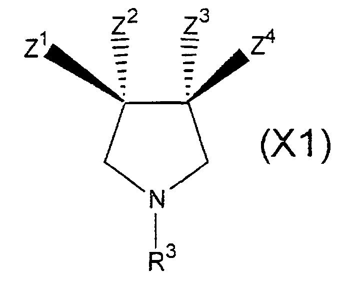 [wherein each of Z¹, Z², Z³ and Z⁴ independently means a hydrogen atom, a halogen atom, a hydroxyl group or a group represented by formula -O-R¹⁰ (wherein R¹⁰ means C₁₋₆ alkyl groups which may have 1 to 3 substituents selected from a substituent group A consisting of a halogen atom, a hydroxyl group, a methoxy group, a phenyl group, C₃₋₈ cycloalkyl groups and C₁₋₆ alkoxy groups, or C₃₋₈ cycloalkyl groups which may have 1 to 3 substituents selected from the substituent group A); any one of Z¹ and Z², and any one of Z³ and Z⁴ mean hydrogen atoms; and R³ means a benzyl group which may have 1 to 3 methoxy group(s) or nitro group(s) as substituent(s), or a hydrogen atom; with the proviso that a case (1) where a group which is not a hydrogen atom in Z¹ and Z² is the same with a group which is not hydrogen atom in Z³ and Z⁴, and a case (2) where a group which is not a hydrogen atom in Z¹ and Z² is a hydroxyl group, and a group which is not a hydrogen atom in Z³ and Z⁴ is an ethoxy group or a chlorine atom are excluded]; or a salt thereof or a hydrate thereof;
<3> The compound described in the above-mentioned item <2>, wherein each of Z¹ and Z⁴ represents a hydrogen atom; or the salt thereof or the hydrate thereof;
<4> The compound described in the above-mentioned item <2>,
   wherein each of Z¹ and Z³ represents a hydrogen atom; or the salt thereof or the hydrate thereof;
<5> The compound described in the above-mentioned item <2>, wherein each of Z² and Z⁴ represents a hydrogen atom; or the salt thereof or the hydrate thereof;
<6> The compound described in the above-mentioned item <1>, wherein R¹ represents a group represented by formula -OR¹⁰ (R¹⁰ has the same meaning as mentioned in item <1>); or the salt thereof or the hydrate thereof;
<7> The compound described in anyone of the above-mentioned items <2> to <5>, wherein at least one of Z¹ and Z² represents a group represented by formula -OR¹⁰ (R¹⁰ has the same meaning as mentioned in item <1>); or the salt thereof or the hydrate thereof;
<8> The compound described in anyone of the above-mentioned items <1> to <7>, wherein R³ represents a hydrogen atom or a benzyl group; or the salt thereof or the hydrate thereof;
<9> The compound described in anyone of the above-mentioned items <1> to <7>, wherein R³ represents a hydrogen atom; or the salt thereof or the hydrate thereof;
<10> The compound described in anyone of the above-mentioned items <1> to <9>, wherein R¹⁰ represents a hydroxy group, a methoxy group or a benzyloxy group; or the salt thereof or the hydrate thereof;
<11> The compound described in the above-mentioned item <1> or <2>, wherein the compound is one compound selected from the group consisting of (3R,4R)-3-hydroxy-4-methoxypyrrolidine, (3S,4S)-3-hydroxy-4-methoxypyrrolidine, (3R,4S)-3-hydroxy-4-methoxypyrrolidine and (3S,4R)-3-hydroxy-4-methoxypyrrolidine; or the salt thereof or the hydrate thereof;
<12> The compound described in anyone of the above-mentioned items <1> to <11>, wherein the salt thereof is a hydrochloride salt or an oxalate salt; or the salt thereof or the hydrate thereof;
<13> A process for producing 3-hydroxy-4-methoxypyrrolidine (6y) represented by the following formula (6y): 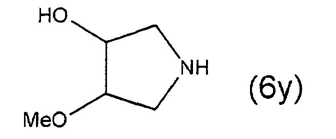 comprising the steps of:
   subjecting a compound (1y) represented by formula (1y) to oxidation reaction to obtain a compound (2y) represented by formula (2y); 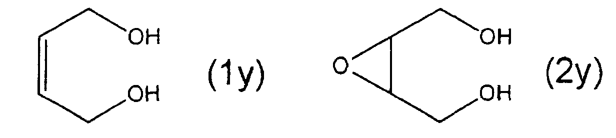 then, converting two hydroxyl groups in said compound (2y) into leaving groups to obtain a compound (3y) represented by formula (3y) (wherein L¹ means a halogen atom, a methylsulfonyloxy group, a p-toluenesulfonyloxy group, and a methanesulfonyloxy group) ; 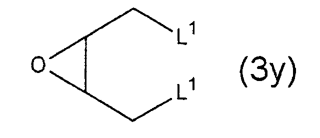 then, allowing said compound (3y) to react with amine derivative represented by general formula R^{3a}-NH₂ (wherein R^{3a} means a benzyl group which may have 1 to 3 methoxy group (s) or nitro group (s) as substituent(s)) to obtain a compound (4y) represented by formula (4y) (wherein R^{3a} has the same meaning as defined above) ; 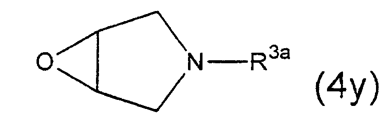 then, allowing said compound (4y) to react with alkaline metal methoxide salt to obtain a compound (5y) represented by formula (5y) (wherein R^{3a} has the same meaning as defined above); and 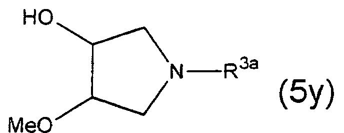 then, releasing R^{3a} in said compound (5y); or a salt thereof or a hydrate thereof;
<14> A process for producing 3-hydroxy-4-methoxypyrrolidine (6y) represented by the following formula (6y): 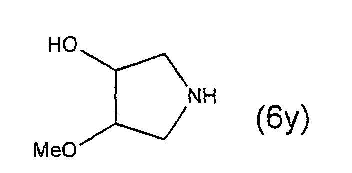 comprising the steps of:
   allowing a compound (4y) represented by formula (4y) (wherein R^{3a} means a benzyl group which may have 1 to 3 methoxy group(s) or nitro group (s) as subs tituent (s)) to react with alkaline metal methoxide salt to obtain a compound (5y) represented by formula (5y) (wherein R^{3a} has the same meaning as defined above); and 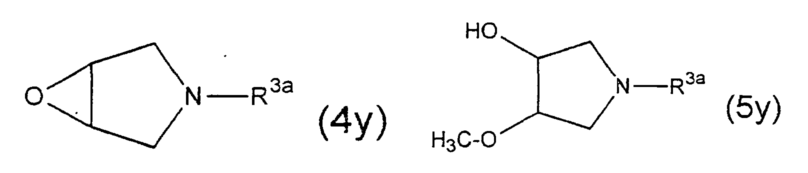 then, releasing R^{3a} in said compound (5y) ; or a salt thereof or a hydrate thereof.
<15> A process for producing 3-hydroxy-4-methoxypyrrolidine (8z) represented by formula (8z): 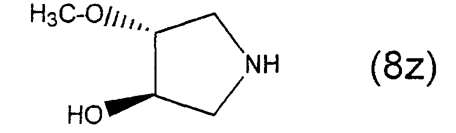 comprising the steps of:
   allowing a compound (1z) represented by the following formula (1z) (wherein R⁵ means C₁₋₆ alkyl groups, a benzyl group or a hydrogen atom) to react with a compound represented by formula Ar-CHO (wherein Ar means a phenyl group which may have 1 to 3 substituent(s) selected from the group consisting of a nitro group and a methoxy group) to obtain a compound (2z) represented by formula (2z) (wherein Ar and R⁵ have the same meanings as defined above); 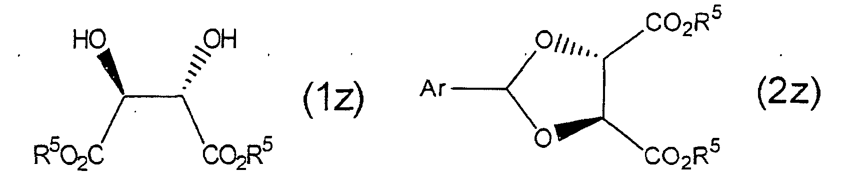 then, subjecting said compound (2z) to reductive reaction to obtain a compound (3z) represented by formula (3z) (wherein Ar has the same meaning as defined above); 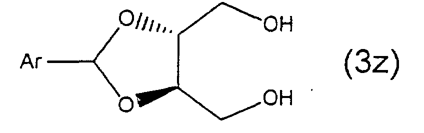 then, converting hydroxyl groups in said compound (3z) into leaving groups to obtain a compound (4z) represented by formula (4z) (wherein Ar has the same meaning as defined above, and L¹ means a halogen atom, a methanesulfonyloxy group, a p-toluenesulfonyl group or a trifluoromethylsulfonyloxy group); 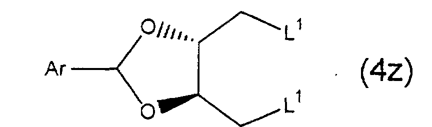 then, subjecting said compound (4z) to reductive reaction to obtain a compound (5z) represented by formula (5z) (wherein Ar and L¹ have the same meanings as defined above); 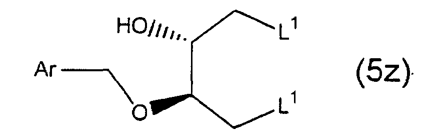 then, allowing said compound (5z) to react with a compound represented by formula R^{3a}-NH₂ (wherein R^{3a} means a benzyl group which may have 1 to 3 methoxy group(s) or nitro group(s) as substituent (s)) to obtain a compound (6z) represented by formula (6z) (wherein Ar and R^{3a} have the same meanings as defined above) ; 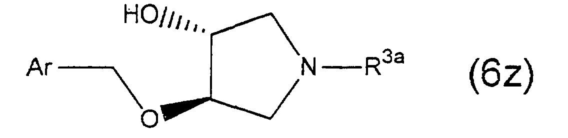 then, subjecting said compound (6z) to methylating reaction to obtain a compound (7z) represented by formula (7z) (wherein Ar and R^{3a} have the same meanings as defined above); and 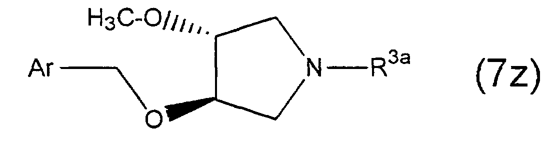 then, subjecting protecting group in said compound (7z) to deprotection; or a salt thereof or a hydrate thereof.
<16> A process for producing 3-hydroxy-4-methoxypyrrolidine (8z) represented by formula (8z): 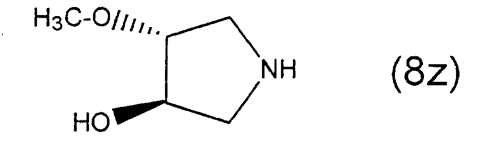 comprising the steps of:
   subjecting a compound (4z) represented by formula (4z) (wherein Ar means a phenyl group which may have 1 to 3 substituent(s) selected from the group consisting of a nitro group and a methoxy group, and L¹ means a halogen atom, a methanesulfonyloxy group, a p-toluenesulfonyl group or a trifluoromethylsulfonyloxy group) to reductive reaction to obtain a compound (5z) represented by formula (5z) (wherein Ar and L¹ have the same meanings as defined above); 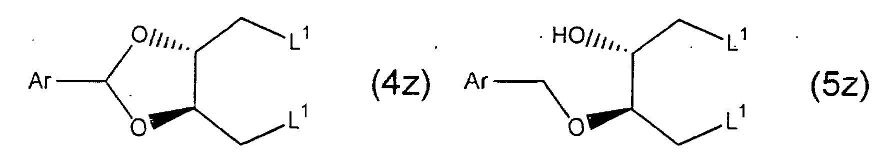 then, allowing said compound (5z) to react with a compound represented by formula R^{3a}-NH₂ (wherein R^{3a} means a benzyl group which may have 1 to 3 methoxy group(s) or nitro group(s) as substituent (s)) to obtain a compound (6z) represented by formula (6z) (wherein Ar and R^{3a} have the same meanings as defined above) ; 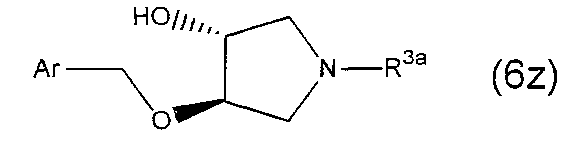 then, subjecting said compound (6z) to methylating reaction to obtain a compound (7z) represented by formula (7z) (wherein Ar and R^{3a} have the same meanings as defined above); and 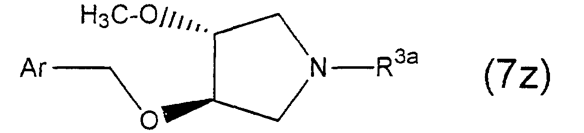 then, subjecting protecting group in said compound (7z) to deprotection; or a salt thereof or a hydrate thereof.

### Preferred Embodiments for Carrying Out the Invention

Hereinafter, meanings of terms, symbols and the like described hereinafter will be defined and the present invention will be described in detail.

In the present specification and claims, a structural formula of a compound may conveniently represent a given isomer. Compounds of the present invention may be isomers thereof such as all geometrical isomers generated from the structure of the compounds, optical isomers based on an asymmetric carbon thereof, stereoisomers and enantiomers, and mixtures of some of the isomers. Accordingly, the compounds of the present invention are not limited to those defined by the structural formula thereof, and may be any one of the isomers, or a mixture of some of the isomers. Some compounds of the present invention may have an asymmetric carbon in the molecule thereof, and thus may be optically active substances thereof or racemic bodies thereof. The compounds of the present invention may have crystal polymorphisms, but are not limited to the crystal form thereof. The compounds of the present invention may be in a single crystal form or in a mixed crystal form. Furthermore, the compounds of the present invention may be in a dehydrate form or in a hydrate form.

In the present specification and claims, the term "C₁₋₆ alkyl group" means a linear or branched alkyl group having 1 to 6 carbon atoms. Specific examples thereof may include methyl, ethyl, n-propyl, i-propyl, n-butyl, i-butyl, t-butyl, n-pentyl, i-pentyl, neopentyl, n-hexyl, 1-methylpropyl, 1,2-dimethylpropyl, 2-ethylpropyl, 1-methyl-2-ethylpropyl, 1-ethyl-2-methylpropyl, 1,1,2-trimethylpropyl, 1-methylbutyl, 2-methylbutyl, 1,1-dimethylbutyl, 2,2-dimethylbutyl, 2-ethylbutyl, 1,3-dimethylbutyl, 2-methylpentyl, 3-methylpentyl groups and the like.

In the present specification and claims, the term "C₃₋₈ cycloalkyl group" means a cyclic aliphatic hydrocarbon group having 3 to 8 carbon atoms. Specific examples thereof may include cyclopropyl, cyclobutyl, cyclopentyl, cyclohexyl, cycloheptyl, cyclopropenyl, cyclobutenyl, cyclopentenyl, cyclohexenyl, cycloheptenyl groups and the like.

In the present specification and claims, the term "halogen atom" means a fluorine, chlorine, bromine or iodine atom.

In the present specification and claims, the term "C₁₋₆ alkoxy group" means an oxy group to which the above-defined "C₁₋₆ alkyl group" is bonded. Specific examples thereof may include methoxy, ethoxy, n-propoxy, i-propoxy, n-butoxy, i-butoxy, sec-butoxy, t-butoxy, n-pentyloxy, i-pentyloxy, sec-pentyloxy, t-pentyloxy, neopentyloxy, 1-methylbutoxy, 2-methylbutoxy, 1,1-dimethylpropoxy, 1,2-dimethylpropoxy, n-hexyloxy, i-hexyloxy, 1-methylpentyloxy, 2-methylpentyloxy, 3-methylpentyloxy, 1,1-dimethylbutoxy, 1,2-dimethylbutoxy, 2,2-dimethylbutoxy, 1,3-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy, 1-ethyl-2-methylpropoxy groups, and the like.

The salt referred to in the present invention may include, but is not limited to, inorganic salts such as hydrofluorides, hydrochlorides, sulfates, nitrates, perchlorates, phosphates, carbonates, bicarbonates, hydrobromates, hydroiodides and the like; salts of organic carboxylic acids such as acetates, maleates, fumarates, oxalates, lactates, tartrates, trifluoroacetates and the like; organic sulfonates such as methanesulfonates, trifluoromethanesulfonates, ethanesulfonates, hydroxymethanesulfonates, hydroxyethanesulfonates, benzenesulfonates, toluenesulfonates, taurine salts and the like; amine salts such as trimethylamine salts, triethylamine salts, pyridine salts, procaine salts, picoline salts, dicyclohexylamine salts, N,N'-dibenzylethylenediamine salts, N-methylglucamine salts, diethanolamine salts, triethanolamine salts, tris(hydroxymethylamino)methane salts, phenetylbenzylamine salts and the like; alkali metal salts such as sodium salts, potassium salts and the like; alkali earth metal salts such as magnesium salts, calcium salts and the like; and amino acid salts such as arginine salts, lysine salts, serine salts, glycine salts, aspartate salts, glutamate salts and the like. Preferably, pharmaceutically acceptable salts are used.

Pyrrolidine derivatives (X) represented by the above-described general formula, compounds according to the present invention, or each optical isomer of the pyrrolidine derivatives (X) can be synthesized in accordance with conventionally well-known organic chemical reactions and the like. For instance, these compounds can be synthesized in accordance with the following processes. 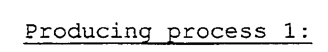 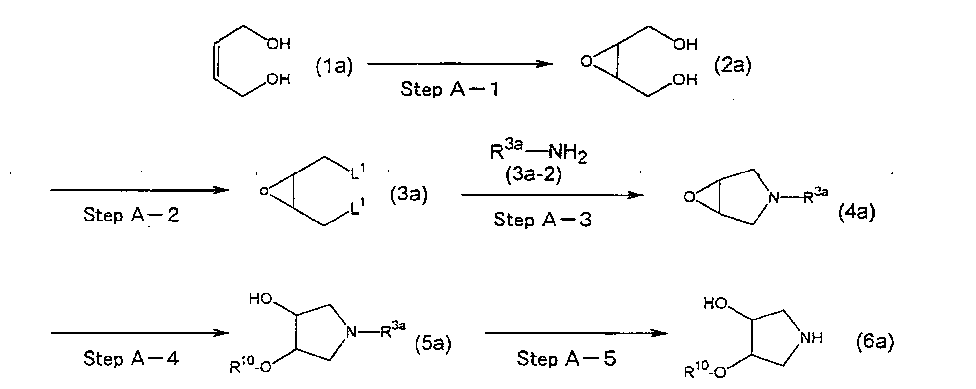 wherein L¹, R^{3a}, and R¹⁰ each has the same meaning as defined above.

### Process A-1, from (1a) to (2a)

The process is one for producing epoxides based on oxidation reaction. The compound (1a), 2-butene-1,4-diol is allowed to react with an oxidizing agent, to obtain the compound (2a). For an oxidizing agent, a peroxy acid such as m-chloroperbenzoic acid (mCPBA) may be used. The reaction is carried out with a reaction solvent including methylene chloride, chloroform, benzene, hexane, t-butanol or the like. A reaction temperature ranges from 0 °C to room temperature.

### Process A-2, from (2a) to (3a)

The process is one for converting hydroxyl group into sulfonate ester group wherein the compound (2a) is allowed to react with a sulfonate esterification reagent to obtain the compound (3a).

The sulfonate esterification reagent includes sulfonic acid halides such as mesyl chloride, tosylate chloride, and tosylate fluoride; sulfonic acid anhydrides such as methanesulfonic acid, tosyl acid anhydride, and trifluoromethanesulfonic acid anhydride; and the like compounds. During the reaction, a base such as triethylamine, N-methyl morpholine, and pyridine may be added. The reaction may be carried out with a reaction solvent including acetone, esters such as ethyl acetate; ethers such as tetrahydrofuran and t-butyl ether; lower nitriles such as acetonitrile; and aprotic solvents such as dimethylformamide, dimethyl sulfoxide and toluene. A reaction temperature ranges from -20 °C to room temperature.

### Process A-3, from (3a) to (4a)

The process is one for amination reaction in accordance with nucleophilic substitution reaction. The compound (3a) is made to react with the compound (3a-2) to obtain the compound (4a). The reaction may be carried out in the presence of a nitrogen-containing base such as triethylamine, N-methyl morpholine, and pyridine or an inorganic base such as sodium carbonate, potassium carbonate, and sodium hydrogen carbonate. A reaction may be carried out with a reaction solvent including acetone; esters such as ethyl acetate; ethers such as tetrahydrofuran and t-butyl ether; lower nitriles such as acetonitrile; aprotic solvents such as dimethylformamide, dimethyl sulfoxide and toluene. A reaction temperature ranges from 0 °C to 80 °C, and preferably around 20 °C to 50 °C. A reaction time is ten hours or longer, and usually forty eight hours are required.

### Process A-4, from (4a) to (5a)

The process is one for allowing the compound (4a) to react with a nucleophilic reagent to obtain the compound (5a).

For a nucleophilic reagent, an alkoxide of alkaline metal represented by formula M-OR¹⁰ (wherein M is sodium, potassium, lithium, magnesium or hydrogen atom and the like; and R¹⁰ has the same meaning as defined above) may be used. A specific example may include sodium methoxide, lithium methoxide and the like. A reaction solvent includes a solvent such as methanol, diethyl ether, tetrahydrofuran, dimethyl sulfoxide, toluene and the like, or a mixed solvent prepared by mixing properly these solvents with each other. A reaction temperature ranges from room temperature to 40 °C, and a reaction time requires usually around several hours.

An alkoxide having formula M-OR¹⁰ obtained by allowing an alcohol represented by formula HO-R¹⁰ (wherein R¹⁰ has the same meaning as defined above) to react with a base may also be used as a nucleophilic reagent. An example of the base includes organic Grignard reagents such as methyl magnesium bromide and i-propyl magnesium bromide; organic lithium reagents such as n-butyl lithium, methyl lithium and phenyl lithium; lithium diisopropyl amide (LDA), sodium hydride, potassium-t-butoxide, lithium bistrimethylsilyl amide, sodium bistrimethylsilyl amide, lithium 2, 2, 6, 6-tetramethylpiperide (LTMP) and the like. For a reaction solvent, methanol, diethyl ether, tetrahydrofuran, dimethyl sulfoxide, toluene or the like; or a mixed solvent prepared by admixing properly these solvents with each other may be used. A reaction temperature ranges from room temperature to a heating reflux temperature, and a reaction time requires usually around several hours.

### Process A-5, from (5a) to (6a)

The process is one wherein the compound (5a) is subjected to elimination reaction of benzyl group to obtain the compound (6a).

The reaction is carried out under the hydrogen atmosphere of a pressure ranging from normal pressures to five atmospheres. The reaction is effected with addition of a catalyst selected from palladium catalysts such as palladium/activated carbon, and palladium hydroxide. Examples of reaction solvent may include those which do not react with hydrogen, i.e. alcohols such as methanol, ethanol and propanol; esters such as ethyl acetate; and ethers such as tetrahydrofuran and t-butyl ether. The reaction is carried out at a reaction temperature ranging from 0 °C to 100 °C, and a reaction time requires usually several hours. In order to take out the compound in the form of a salt, an inorganic acid such as hydrochloric acid or an organic acid such as acetic acid and oxalic acid may be added. 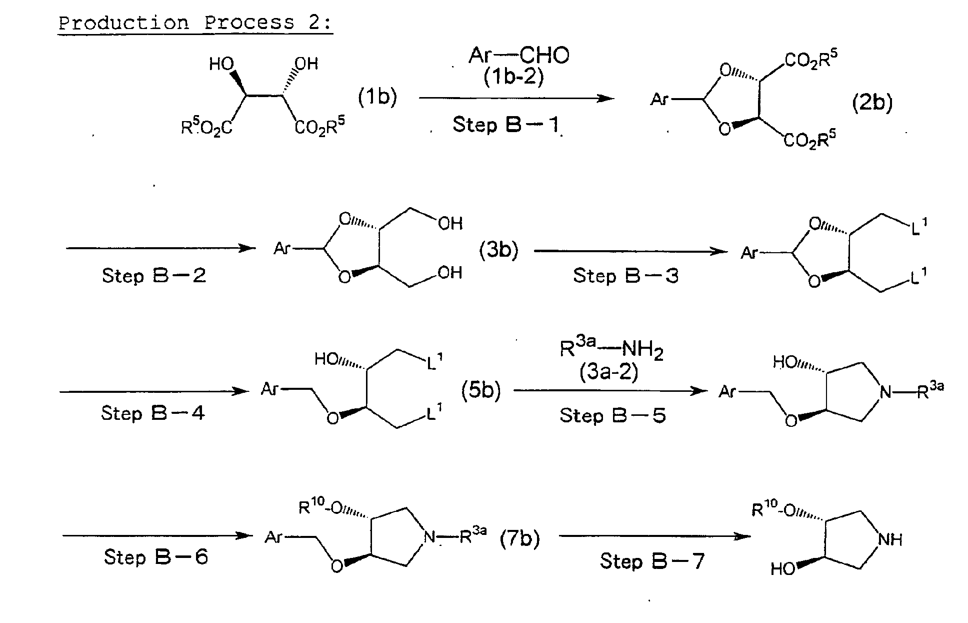 wherein the respective symbols mean the same groups as defined above.

### Process B-1, from (1b) to (2b)

The process is one for acetalization reaction wherein the compound (1b is allowed to react with the compound (1b-2 to obtain the compound (2b). The reaction may be carried out with addition of an acid catalyst such as toluene sulfonic acid, and camphor sulfonic acid as well as an orthoformate compound such as ethyl orthoformate. A reaction solvent includes a solvent such as toluene and benzene. The reaction is carried out usually at 70 °C to a heating reflux temperature, and it is preferable to remove alcohol during the reaction. A reaction time requires usually several hours.

### Process B-2, from (2b) to (3b)

The process is one for reductive reaction wherein the compound (2b) of an ester is made to react with a reducing agent to obtain the compound (3b) of an alcohol. For a reducing agent, for example, sodium borohydride (NaBH₄), lithium borohydride (LiBH₄), sodium cyanoborohydride (NaBH₃CN), aluminum sodio-bis(2-methoxyethoxy)hydride (Red-Al) or the like may be used. For a solvent, any of lower alcohols such as methanol and ethanol; tetrahydrofuran; or toluene, or a mixed solution prepared by admixing properly these solvents may be used. The reaction is carried out at a temperature of from 0 °C to room temperature, and the reaction proceeds for a comparatively short period of time, i.e. it completes within several hours.

### Process B-3, from (3b) to (4b)

The process is one for converting hydroxyl group(s) into leaving group(s). The reaction may be implemented in the same condition as that of the above-described <Process A-2>.

### Process B-4, from (4b) to (5b)

The process is one for making the compound (4b) to react with a reducing agent to obtain the compound (5b). For a reducing agent, a material prepared by combining lithium aluminum hydride (LiAlH₄), aluminum diisobutylhydride (DIBAL-H), or sodium borohydride (NaBH₄) with a proton acid such as hydrochloric acid, sulfuric acid, and trifluoroacetic acid, or Lewis acid such as trifluoroborane-diethyl ether complex (BF₃Et₂O); or borane (BH₃) or the like may be used. A reaction solvent includes aprotic solvent such as tetrahydrofuran, dimethoxyethane, ethyl acetate and acetonitrile. The reaction is made at a temperature of from -20 °C to room temperature. A reaction time requires usually several hours.

### Process B-5, from (5b) to (6b)

The process is one for amination reaction according to nucleophilic substitution reaction. The reaction may be effected in the same condition as that of the above-described <Process A-3>.

### Process B-6, from (6b) to (7b)

The process is one wherein hydroxy group of the compound (6b) is subjected to alkylation to obtain the compound (7b). The reaction is carried out with addition of a base and an alkylating reagent. For a base, sodium hydrate, potassium hydrate, butyl lithium or the like may be used. The alkylating reagent means a compound represented by a formula U¹-R¹⁰ (wherein U¹ represents a halogen atom, a methylsulfonyloxy group, a p-toluenesulfonyloxy group or the like, and R¹⁰ has the same meaning as defined above). Specific examples of the compound may include methyl halides, sulfonic methyl esters and the like such as methyl iodide, methyl bromide, methanesulfonic methyl ester and toluenesulfonic methyl ester.

It is preferable to implement the reaction in such that the compound (6b) is allowed to react with a base to produce an alkoxide salt of alkaline metal, and then, an alkylating reagent is added thereto to keep the reaction.

A reaction solvent, which does not react with a strong base, may be employed. Example thereof may include tetrahydrofuran, dimethoxyethane, toluene or the like. The reaction is carried out at a temperature of from -78 °C to room temperature, and a reaction time requires around several hours.

### Process B-7, from (7b) to (8b)

The process is one for eliminating benzyl group and the like. The reaction may be carried out in the same condition as that of the above-described <Process 5>. 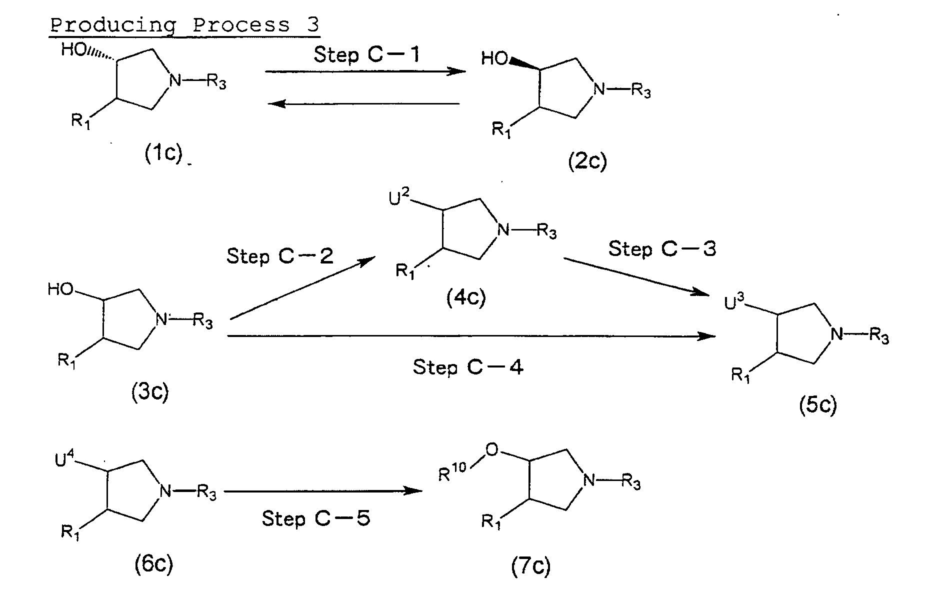 wherein each of R¹, R³ and R¹⁰ has the same meaning as defined above; U² means a methanesulfonyloxy group, a p-toluenesulfonyloxy group, a trifluoromethanesulfonyloxy group or the like; U³ means a halogen atom; and U⁴ means a halogen atom, a methanesulfonyloxy group, a p-toluenesulfonyloxy group, or a trifluoromethanesulfonyloxy group.

### (Process C-1)

The process is one for stereoinversion reaction of hydroxy group according to Mitsunobu reaction wherein the compound (1c) is made to react with a phosphine compound, an azodicarboxylate compound, and a nucleophilic reagent, whereby the compound (2c) can be obtained. The compound (1c) can be obtained from the compound (2c) in a manner similar to thereto.

Examples of phosphine compounds may include triphenylphosphine, tributylphosphine and the like; and examples of azodicarboxylate compounds may include diethylazodicarboxylate, diisopropylazodicarboxylate and the like. For the nucleophilic reagent, formic acid, benzoic acid or the like may be used. The reaction is carried out in a solvent of tetrahydrofuran or the like at room temperature.

### (Process C-2)

The process is one for converting a hydroxyl group into a sulfonate ester group. The reaction may be conducted in the same condition as that of the above-described <Process A-2>.

### (Process C-4)

The process is one for converting a hydroxyl group into a halogen atom wherein the compound (3c) is allowed to react with a halogenating reagent to obtain the compound (5c).

For a halogenating reagent, carbon tetrachloride, N-chlorosuccinimide, phosphorus oxychloride, thionyl chloride, carbon tetrabromide, N-bromosuccinimide, bromine, phosphorus tribromide, phosphorus pentabromide, iodine, diethylaminosulfur trifluoride (DAST), sulfur tetrafluoride, morpholinosulfur trifluoride or the like may be employed. The reaction may be carried out with proper addition of a base such as triethylamine, imidazole and dimethylaminopyridine, or a phosphorus compound such as triphenylphosphine to the reaction solution. For a reaction solvent, carbon tetrachloride, dichloromethane, benzene, toluene, diglyme, isooctane, monofluorotrichloromethane or the like may be employed. The reaction is carried out at -80 °C to heating reflux condition, and it is preferable that a reaction temperature ranges from - 80 °C to room temperature in case of fluorination, while it is preferable that the reaction temperature ranges from room temperature to heating reflux temperature in case of chlorination, bromination or iodination.

### (Process C-3)

The process is one for converting sulfonate ester into a halogen atom wherein the compound (4c) is allowed to react with a halogenating reagent to obtain the compound (5c). For the halogenating agent, lithium chloride, sodium bromide, tris (dimethylamino) sulfonium difluorotrimethylsilicate (TASF), potassium fluoride, tetrabutylammonium fluoride (TBAF) or the like may be employed. For a reaction solvent, dichloromethane, benzene, toluene, dimethylformamide or the like may be used, and a reaction temperature ranges from room temperature to heating reflux temperature.

### (Process C-5)

The process is one for making the compound (6c) to react with a nucleophilic reagent to obtain the compound (7c). The reaction may be carried out in the same condition as that of the above-described <Process A-4>.

Optical isomers of the respective compounds described in the production process B can be obtained with the use of compounds which are optical isomers of the compound (1b) for a raw material of <Process B-1> having the following formula (1b-3) (wherein R⁵ has the same meaning as defined above). Furthermore, not only the case where the respective compounds relate to racemic bodies, but also the case where they relate to chiral bodies can be applied to a condition of the production process C.

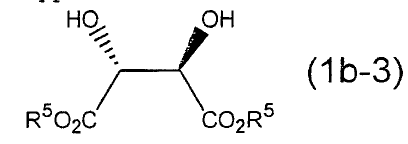

As described above, solvents which may be used in the present invention are any solvent which does not inhibit the reaction and is used commonly for organic synthesis, so that there is no particular limitation. Specific examples of the solvents include lower alcohols such as methanol, ethanol, propanol and butanol; polyalcohols such as ethylene glycol and glycerin; ketones such as acetone, methyl ethyl ketone, diethyl ketone and cyclohexanone; ethers such as diethyl ether, isopropyl ether, tetrahydrofuran, dioxane, 2-methoxyethanol and 1,2-dimethoxyethane; nitriles such as acetonitrile and propionitrile; esters such as methyl acetate, ethyl acetate, isopropyl acetate, butyl acetate and diethyl acetate and diethyl phthalate; halogenated hydrocarbons such as dichloromethane, chloroform, carbon tetrachloride, 1,2-dichloroethane, trichloroethylene and tetrachloroethylene; aromatics such as benzene, toluene, xylene, monochlorobenzene, nitrobenzene, indene, pyridine, quinoline, collidine and phenol; hydrocarbons such as pentane, cyclohexane, hexane, heptane, octane, isooctane, petroleum benzine and petroleum ether; amines such as ethanolamine, diethylamine, triethylamine, pyrrolidine, piperidine, piperazine, morpholine, aniline, dimethylaniline, benzylamine and toluidine; amides such as formamide, N-methylpyrrolidone, N,N-dimethylimidazolone, N,N-dimethylacetamide and N,N-dimethylformamide; phosphoric amides such as hexamethylphosphoric triamide and hexamethylphosphorus triamide; water; and a mixed solvent prepared from one, two or more of these and the other solvents used commonly wherein a mixing ratio thereof is not specifically limited.

As described above, bases which may be used in the present invention are any base which does not inhibit the reaction and is known as base for organic synthesis, so that there is no particular limitation. Specific examples of the bases may include sodium carbonate, sodium hydrogen carbonate, potassium carbonate, sodium hydride, potassium hydride, t-butoxy potassium, pyridine, dimethylaminopyridine, trimethylamine, triethylamine, N,N-diisopropylethylamine, N-methylmorfoline, N-methylpyrrolidine, N-methylpiperidine, N,N-dimetylaniline, 1,8-diazabicyclo[5.4.0]undeca-7-ene (DBU), pyridine, 4-dimethylaminopyridine, picoline, lutidine, quinoline, isoquinoline, sodium hydroxide, potassium hydroxide, lithium hydroxide, butyllithium; sodium or potassium alcoholates such as sodium methylate, potassium methylate and sodium ethylate and the like.

As described above, reducing agents which may be used in the present invention are any material which does not inhibit the reaction and is used commonly for organic synthesis, so that there is no particular limitation. Specific examples of the reducing agents may include sodium borohydride (NaBH₄), lithium borohydride (LiBH₄) , zinc borohydride (Zn(BH₄)₂), tetramethylammonium triacetoxyborohydride (Me₄NBH(OAc)₃), sodium cyanoborohydride (NaBH₃CN), potassium trialkylborohydride (Selectride), lithium triethylborohydride (Super Hydride (LiBHEt₃)), lithium aluminum hydride (LiAlH₄), diisobutyl aluminum hydride (DIBAL), tri(t-butoxy)lithium aluminum hydride (LiAlH(t-BuO)₃), aluminum sodio-bis(2-methoxyethoxy)hydride (Red-Al), aluminum sodio-bis (2-methoxyethoxy) hydride (binap) and the like as well as catalytic hydrogenated substances of platinum, palladium, rhodium, ruthenium, nickel and the like.

After completing the reaction, it is possible to purify a resulting product, if desired, in accordance with a usual treating method, for example, column chromatography wherein silica gel, adsorption resin or the like is employed, or recrystallization by the use of a proper solvent.

### Examples:

The present invention will be demonstrated specifically with reference to the following examples, but it is to be noted that the invention is not limited by these examples. 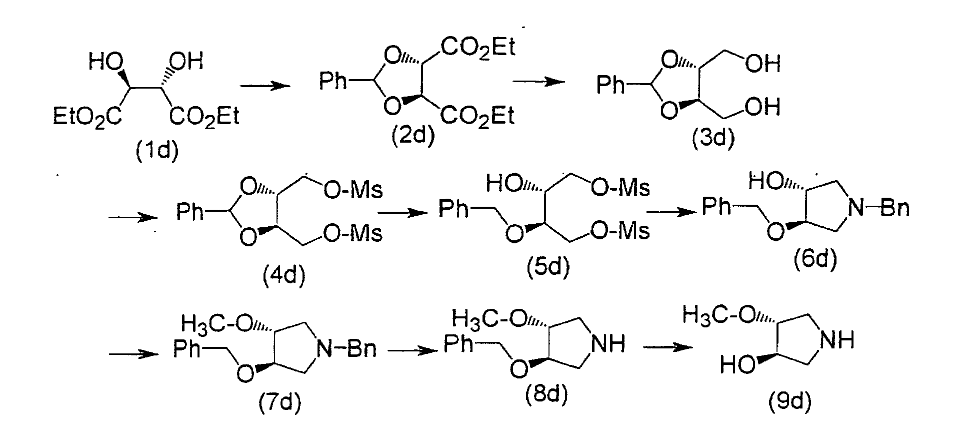 wherein Ph means a phenyl group; Bn means a benzyl group; Et means an ethyl group; and Ms means a methanesulphonyl group.

### Example 1:

### Diethyl(4S,5S)-2-phenyl-1,3-dioxolan-4,5-dicarboxylate (2d)

At room temperature, ethyl orthoformate (74 g, 0.499 mol) , 10-camphor sulfonic acid (1.8 g, 7.749 mmol) and p-toluene sulfonic acid-hydrate (1.48 g, 7.780 mmol) were added to a solution of benzaldehyde (53 g, 0.499 mol) in toluene (300 ml) , and the resulting mixture was subjected to heating reflux. After forty five minutes, a solution of (S,S)-diethyl tartrate (100 g, 0.485 mol) in toluene was further added, and alcohol produced under heating reflux was removed. After the solvent was removed, ethyl acetate (400 ml) was added to the resulting product, followed by washing with a saturated sodium hydrogen carbonate aqueous solution and saturated saline solution, and adding anhydrous magnesium sulfate and activated carbon. After filtration, the solvent was removed, and then precipitated crystal was washed with n-hexane, to obtain 134.3 g (94.1 % yield) of the titled compound.
¹H-NMR(CDCl₃) δ: 1.31 (3H, t, J=7.1 Hz) , 1.36 (3H, t, J=7.1 Hz), 4.27(2H, q, J=7.1Hz ), 4.33 ( 2H, q, J=7.1 Hz), 4 . 83 (1H, d, J=4.2 Hz), 4.95 (1H, d, J=4.2 Hz), 6.15 (1H, s), 7.37-7.47 (3H, m), 7.56-7.64 (2H, m).

### Example 2:

### (4R,5R)-4,5-dihydroxymethyl-2-phenyl-1,3-dioxolan (3d)

A solution of diethyl(4S,5S)-2-phenyl-1,3-dioxolan-4,5-dicarboxylate (2d) (30 g, 0.102 mol) in ethanol (150 ml) was added dropwise to an ice-cold solution of sodium borohydride (6.7g, 0.163 mol) in ethanol (100 ml). Then, the admixture was stirred at room temperature for 1.5 hours. After the solvent was removed, ethyl acetate (200 ml) was added to the admixture. The resulting product was washed with saturated saline solution, and anhydrous magnesium sulfate was added thereto. After filtration, the solvent was removed, to obtain 20.5 g (95. 6 % yield) of the titled compound.
¹H-NMR (CDCl₃) δ:2.29-2.37 (2H, m), 3.74-3.91 (4H, m), 4.17 (2H, s), 6.00 (1H, s), 7.38-7.43 (3H, m), 7.46-7.51 (2H, m).

### Example 3:

### (4R,5R)-4,5-dimethanesulfonyloxymethyl-2-phenyl-1,3-dioxola n (4d)

To an ice-cold solution of (4R,5R)-4,5-dihydroxymethyl-2-phenyl-1,3-dioxolan (3d) (20.5 g, 0.098 mol) in tetrahydrofuran (200ml) was added triethylamine (32 ml, 0.230 mol), then, methanesulfonyl chloride (15.2 ml, 0.196 mol) was added thereto. The admixture was stirred for thirty minutes. To the reaction solution was added ethyl acetate (200 ml) , the mixture was washed with water, a saturated sodium hydrogen carbonate aqueous solution, and saturated saline solution. To the resulting product were added anhydrous magnesium sulfate and activated carbon. After filtration, the solvent was removed, and the precipitated crystal was washed with n-hexane, to obtain 31.33 g (87.7 % yield) of the titled compound.
¹H-NMR(CDCl₃) δ: 3.02 (3H, s),3.12 (3H, s), 4.39-4.47 (6H, m), 6.02 (1H, s), 7.38-7.42 (3H, m), 7.46-7.51 (2H m).

### Example 4:

### (2R,3R)-3-benzyloxy-1,4-dimethanesulfonyloxy-2-hydroxybutan e (5d)

To an ice-cold solution of (4R,5R)-4,5-dimethanesulfonyloxymethyl-2-phenyl-1,3-dioxola n (4d) (31.33 g, 0.085 mol) in acetonitrile (250ml) were added sodium borohydride (8.8 g, 0.214 mol), and then, a 4N hydrochloric acid/ethyl acetate solution (107 ml, 0.428 mol) such that a temperature of the mixture did not reach 10 °C or higher. Then, the mixture was stirred at room temperature for 2.5 hours. The reaction solution was added dropwise to an aqueous solution (200 ml) of ethyl acetate (300 ml)/saturated sodium hydrogen carbonate. Then, an organic layer was washed with saturated saline solution, and anhydrous magnesium sulfate was added thereto. After filtration, the solvent was removed to obtain 31.3 g (99.4 % yield) of the titled compound.
¹H-NMR(CDCl₃) δ:3.03 (3H, s), 3.04 (3H, s), 3.79-3.84 (1H, m), 3.99-4.05 (1H, m), 4.19 (1H, dd, J=11.0, 5.1 Hz), 4.28 (1H, dd, J=11.0, 5 .1 Hz) 4.33 (1H, dd, J=11.0, 5 .1 Hz), 4.44 (1H, dd, J=11.0, 5.1 Hz), 4.60 (1H, d, J=11.6 Hz), 4.77 (1H, d, J=11.6 Hz), 7.29-7.40 (5H, m).

### Example 5:

### (3R,4R-1-benzyl-3-benzyloxy-4-hydroxypyrrolidine(6d)

Under nitrogen stream, benzylamine (83 g, 0.77 mol) was added to (2R,3R)-3-benzyloxy-1,4-dimethanesulfonyloxy-2-hydroxybutane (5d) (28.5 g, 0.077 mol). The mixture was stirred at room temperature for two days. Unreacted benzylamine was removed under reduced pressure, then, ethyl acetate (300 ml) was added to the residue. The resulting product was washed with a saturated sodium hydrogen carbonate aqueous solution and saturated saline solution, and then magnesium sulfate was added thereto. After filtration, the solvent was removed, and the residue was purified with silica column chromatography (developing solution: n-hexane, ethyl acetate), to obtain 18.2 g (83.0 % yield) of the titled compound.
¹H-NMR(CDCl₃) δ: 2.35 (1H dd, J=9.8, 5.7Hz) , 2.65-2.73 (2H, m) , 3.16 (1H, dd, J=10.7, 6.7 Hz), 3.60 (1H, d, J=12.3 Hz), 3.64 (1H, d, J=12.3Hz), 3.90-3.94 (1H, m), 4.17-4.21 (1H, m), 4.51 (1H, d, J=12.0 Hz), 4.57 (1H, d, J=12.0 Hz), 7.25-7.35 (10H, m).

### Example 6:

### (3R,4R)-1-benzyl-3-benzyloxy-4-methoxypyrrolidine (7d)

Under nitrogen stream, a tetrahydrofuran (35 ml) solution of (3R,4R)-1-benzyl-3-benzyloxy-4-hydroxypyrrolidine (6d) (17.5 g, 0.062 mol) was added dropwise to an ice-cold solution of sodium hydride (2.96 g, 0.074 mol) in tetrahydrofuran (70 ml). Then, the mixture was stirred at room temperature for three hours. The reaction solution was cooled to a temperature of -65 °C, then, a solution of methyl iodide (9.0 g, 0.063 mol) in tetrahydrofuran (35 ml) was added dropwise thereto. Then, the mixture was stirred overnight at room temperature. To the reaction solution was added ethyl acetate (100 ml), the mixture was washed with water and saturated saline solution, and magnesium sulfate was added thereto. After filtration, the solvent was removed, and the residue was purified with silica column chromatography (developing solution: n-hexane, ethyl acetate), to obtain 13.06 g (71.1% yield) of the titled compound.
¹H-NMR (CDCl₃) δ: 2.54 (1H, dd, J=9.9, 4.5 Hz), 2 . 58 (1H, dd, J=9.9, 4.5 Hz), 2.83 (1H, dd, J=9.9, 6.2 Hz), 2.94 (1H, dd, J=9.9, 6.2 Hz), 3.31 (3H, s), 3.57 (1H, d, J=12.9 Hz), 3.65 (1H, d, J=12.9 Hz), 3.81-3.86 (1H, m), 3.95-4.00 (1H, m), 4.52 (2H, s), 7.25-7.35 (10H, m).

### Example 7:

### (3R,4R)-3-benzyloxy-4-methoxypyrrolidine (8d)

At room temperature, 3.6 g of palladium/activated carbon (5 wt% palladium on dry weight basis) were added to a solution of (3R,4R)-1-benzyl-3-benzyloxy-4-methoxypyrrolidine (7d) (12.0g, 0.040mol) in methanol (120ml). The mixture was allowed to react with each other overnight under normal pressure and hydrogen atmosphere. The reaction solution was subjected to Celite filtration, and then, the solvent was removed, to obtain (quantitatively) 8.4 g of the titled compound.
¹H-NMR(CDCl₃) δ: 2.87 (2H, dd, J=12.5, 3.1 Hz), 2.93 (1H, dd, J=12.5, 3.1Hz), 3.07-3.17 ( 2H, m) , 3.33 (3H, s), 3.79 (1H, broad s), 3.92 (1H, br-s), 4.54 (2H, s), 7.25-7.35 (5H, m).

### Example 8:

### (3R,4R)-3-hydroxy-4-methoxypyrrolidine hydrochloride (9d)

At room temperature, 2.0 g of palladium hydroxide/carbon (20 wt% palladium on dry weight basis) and 4N hydrochloric acid/ethyl acetate solution (20 ml, 0.080 mol) were added to a solution of (3R,4R)-3-benzyloxy-4-methoxypyrrolidine (8d) (8.4 g, 0.040 mol) in methanol (120 ml). The mixture was allowed to react with each other for three hours under three atmospheric pressure and hydrogen atmosphere. The reaction solution was subjected to Celite filtration, and thereafter, the solvent was removed, to obtain (quantitatively) 6.13 g of the titled compound.
¹H-NMR(D₂O) δ: 3.17 (1H, d, J=12.7 Hz), 3.25 (3H,s), 3.28-3.35 (2H, m), 3.38 (1H, dd.=12.7, 3.9 Hz), 3.89 (1H, d, J=3.9 Hz), 4.38 (1H, d, J=3.9 Hz).

The racemic modification of the present invention can be synthesized by way of the following route. 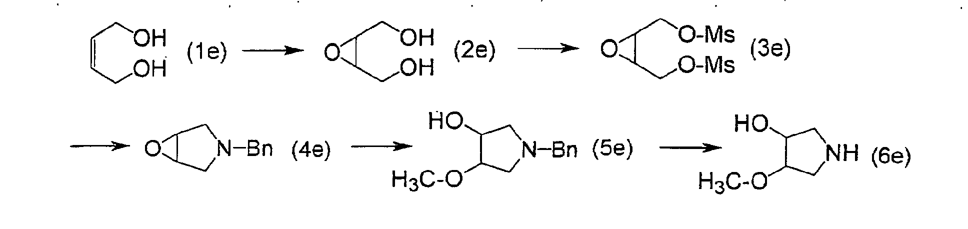 wherein Bn means a benzyl group; and Ms means a methanesulfonyl group.

### Example 9:

### 1-Benzyl-3,4-epoxypyrrolidine (4e)

To 1,4-dimethanesulfonyloxy-3,4-epoxybutane (3e) (29.51 g, 113 mmol) were added 58.5 ml of tetrahydrofuran. A solution prepared by adding 20 ml of tetrahydrofuran to benzylamine (37.0 ml, 3.0eq.) was added dropwise to the resulting mixture on a water bath with stirring. The container therefor was washed clean with 10 ml of tetrahydrofuran. After stirring forty seven hours at room temperature, the reaction solution was poured into 153 ml of 2N sodium hydroxide. The resulting product was extracted twice with 118 ml of t-butyl ether. The upper layer of the solution was dried over anhydrous magnesium sulfate, and dried at 40 °C in vacuo, to obtain 43.8 g of a yellow solution. The resulting crude product was purified with silica gel column chromatography (silica gel: (Wako gel C-200) 526 g, developing solution: t-butylmethyl ether), to obtain 17. 9 g of the titled compound in the form of yellow liquid (90 % yield).
¹H-NMR(400MHz, CD₃CN) d: 2.48 (2H, d, J=11.6 Hz) , 3.03 (2H, d, J=11.6 Hz), 3.55 (2H, s), 3.64 (2H, s,) 7.21-7.32 (5H, m).

### Example 10:

### Trans-1-benzyl-3-hydroxy-4-methoxypyrrolidine (5e)

To a 28 % solution of sodium methoxide in methanol (15.2 g, 78.6 mmol) was added 1-benzyl-3,4-epoxypyrrolidine (4e) (13.8 g, 78.6 mmol). The container therefor was washed with 4 ml of methanol. After stirring the mixture at 70°C for three hours, 69 ml of ethyl acetate and 69 ml of water were added to the reaction solution. After separation of liquids, the aqueous layer was extracted again with 69 ml of ethyl acetate, and the resulting layer was admixed with the upper layer. The organic layer was dried over anhydrous magnesium sulfate, and dried in vacuo at 40 °C, to obtain 16.0 g of orange liquid. The resulting crude product was purified with silica gel column chromatography (silica gel (Wako gel C-200) 192 g, developing solution: t-butylmethyl ether → 2% methanol/ethyl acetate), to obtain 12.4 g (76 % yield) of the titled compound in the form of yellowish orange liquid.
¹H-NMR(400MHz, CD₃CN) d : 2.34-2.38 (2H, m), 2.74 (1H, dd, J=6.1, 9.8 Hz), 2.84 (1H, dd, J=10.0, 6.6 Hz), 3.26 (3H, s), 3.51 (1H, d, J=12.8 Hz), 3.57 (1H, d, J=12.8 Hz), 3.57-3.60 (1H, m), 3.99-4.03 (1H, m), 7.22-7.33 (5H, m).

### Example 11:

### Trans-3-hydroxy-4-methoxypyrrolidine (6e)

At room temperature, palladium hydroxide/carbon (20 wt% palladium) (1.2 g) were added to a solution of trans-l-benzyl-3-hydroxy-4-methoxypyrrolidine (5e) (12 g, 0.058 mol) in methanol (72 ml). The mixture was allowed to react with each other for three hours under three atmospheric pressure and hydrogen atmosphere. The reaction solution was subjected to Celite filtration, and thereafter, the solvent was removed under reduced pressure, to obtain (quantitatively) 6.8 g of the titled compound.
¹H-NMR(400MHz, CD₃CN) d: 2.78 (1H, d, J=11.9 Hz), 2.85 (1H, dd, J=11.9, 2.0 Hz), 3.05 (2H, broad s), 3.12 1H, dd, J=11.9, 5.0 Hz), 3.36 (3H, s), 3.65 (1H, d, J=5.0 Hz), 4.18 (1H, d, J=5.0 Hz).

### Example 2-1:

### (6-(3R,4R)-3-hydroxy-4-methoxypyrrolidine)-2-benzylpyridine

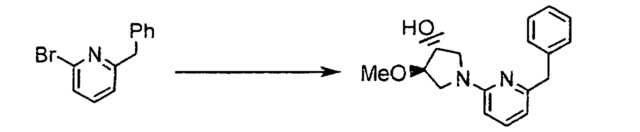

In the atmosphere of nitrogen, to a 2L four necked flask were added (3R,4R)-3-hydroxy-4-methoxypyrrolidine (106 g, 0. 91 mol), 6-bromo-2-benzylpyridine (150 g, 0.605 mol) [see Tetrahedron Letters, 21, pp. 845-848 (1980)], 1,8-diazabicyclo[5.4.0]-undeca-5-ene (92 g, 0.605 mol) and N-methylpyrrolidone (150 mL). Then, under stirring, the solution was heated in an oil bath at 110 °C for 11 hours. (3R,4R)-3-hydroxy-4-methoxypyrrolidine (10.6 g, 0.09 mol) was added thereto. Under stirring, the solution was heated in an oil bath of 110 °C for 1 hour. The solution was left at room temperature, and then thereto were added 450 mL of t-butyl methyl ether and 450 mL of water. While the temperature of the inside was kept at 20 °C or less by an ice bath, 2 N hydrochloric acid was dropwise added to the solution until the pH of the solution was 6. The solution was transferred to a 2 L separatory funnel. The upper layer was separated, and the remaining aqueous layer was again subjected to extraction with 300 mL of t-butyl methyl ether. The t-butyl methyl ether layers were combined, followed by washing with 300 mL of water and 300 mL of saturated saline solution. The upper layer was dried over anhydrous magnesium sulfate, and washed with 100 mL of t-butyl methyl ether. The filtrate was concentrated under reduced pressure at 40 °C to give 171 g (crude yield: 99.6 %) of the captioned compound as a black brown oily material.
¹H-NMR(400MHz, CDCl₃) δ: 3.42 (3H, s), 3.49 (1H, dd, J=3, 12 Hz), 3.52 (1H, dd, J=3, 12 Hz), 3.71 (1H, dd, J=5, 12 Hz), 3.75 (1H, dd, J=5, 12 Hz), 3.85-3.88 (1H, m) , 3 . 97 (2H, s), 4.38-4.40 (1H, m), 6.16(1H, d, J=8 Hz), 6.35(1H, d, J=8 Hz), 7.17-7.34 (6H, m).

### Example 2-2:

### (6-(3R,4R)-3-hydroxy-4-methoxypyrrolidine-1-yl)-3-bromo-2-benzylpyridine

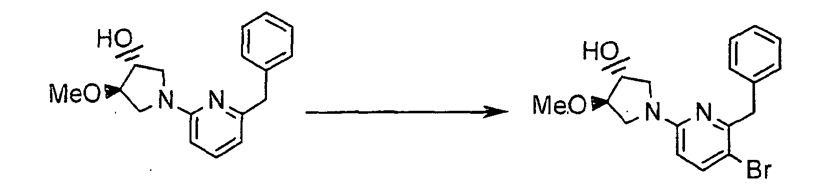

A solution of (6-(3R,4R)-3-hydroxy-4-methoxypyrrolidine-1-yl)-2-benzylpyr idine (2 g, 7 mmol) in N,N-dimethylformamide (8 mL) was stirred in an ice bath. N-bromosuccinimide (1.3 g, 7.4 mol) was added little by little to the solution. Thereto was added t-butyl methyl ether (100 mL) to separate the solution into two layers. The upper layer was washed with 100 mL of an aqueous 5% sodium thiosulfate solution, and saturated saline solution. The layer was dried over anhydrous magnesium sulfate, concentrated under reduced pressure, and purified by silica gel chromatography (developing solution: hexane : ethyl acetate = 3:1) to yield 2.1g (yield: 83%) of the captioned compound as an oily material.
¹H-NMR(400MHz, CDCl₃) δ: 3.41(3H, s), 3.42-3.50 (2H, m), 3.64-3.71 (2H, m), 3.84-3.86 (1H, m), 4.15 (2H, s), 4.39-4.41 (1H, m), 6.09 (1H, d, J=9 Hz), 7.18 (1H, dd, J=8, 8 Hz), 7.26 (2H, dd, J=8, 8 Hz), 7.36 (2H, d, J=8 Hz), 7.48 (1H, d, J=8 Hz).

### Example 2-3:

### (3R)-3-[2-benzyl-6-((3R,4R)-3-hydroxy-4-methoxypyrrol idine-1-yl]pyridine-3-yl]ethynyl)quinuclidine-3-ol

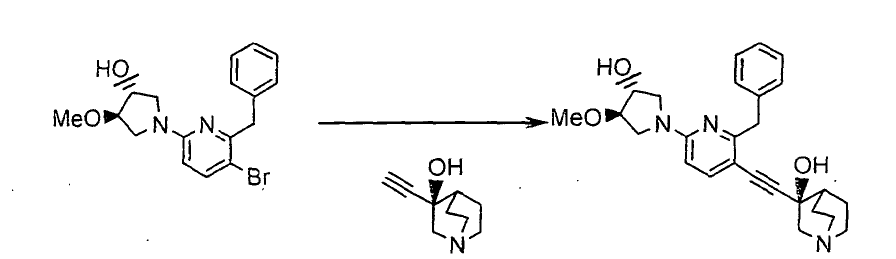

(6-(3R,4R)-3-hydroxy-4-methoxypyrrolidine-1-yl)-3-bro mo-2-benzylpyridine (1.14 g, 3 mmol), (3R)-quinuclidine-3-ol (680 mg, 4.5 mmol), and triphenylphosphine (79 mg, 0.3 mmol) were dissolved in 10 mL of piperidine. Thereto were added palladium tetrakis(triphenylphosphine) (173mg, 0.15 mmol) and copper iodide (57 mg, 0.3 mmol) , and then the solution was heated at 100 °C for 5 hours. To the reaction solution were added tetrahydrofuran and ethyl acetate, and the resultant solution was washed with water. Thereafter, anhydrous magnesium sulfate was added to the solution so as to dry the solution. The filtrate was concentrated under reduced pressure to yield an oily material containing the captioned compound.

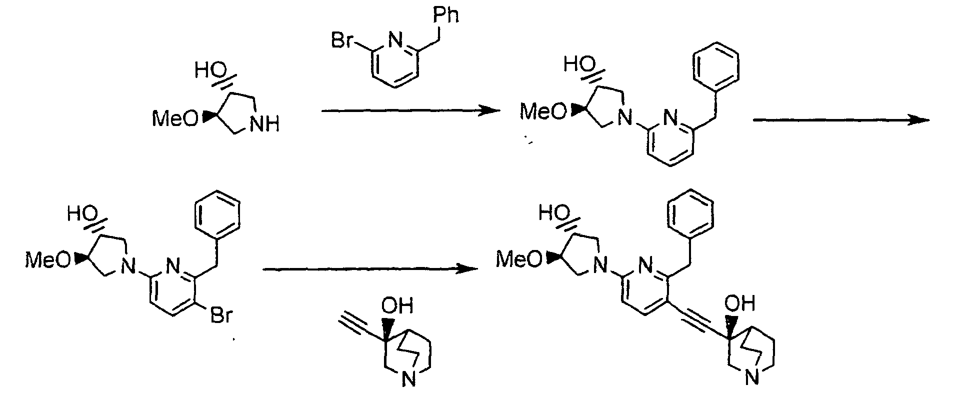

## Claims

1. A compound represented by the general formula (X); 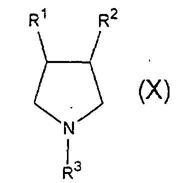 [wherein each of R¹ and R² independently means a halogen atom, a hydroxyl group or a group represented by formula -O-R¹⁰ (wherein R¹⁰ means C₁₋₆ alkyl groups which may have 1 to 3 substituents selected from a substituent group A consisting of a halogen atom, a hydroxyl group, a methoxy group, a phenyl group, C₃₋₈ cycloalkyl groups and C₁₋₆ alkoxy groups, or C₃₋₈ cycloalkyl groups which may have 1 to 3 substituents selected from the substituent group A; and R³ means a benzyl group which may have 1 to 3 methoxy group (s) or nitro group (s) as substituent (s), or a hydrogen atom; with the proviso that a case (1) where R¹ and R² are the same with each other, and a case (2) where either of R¹ and R² is a hydroxyl group, and another is an ethoxy group or a chlorine atom are excluded); or a salt thereof or a hydrate thereof.

2. A compound represented bv the general formula (Xl); 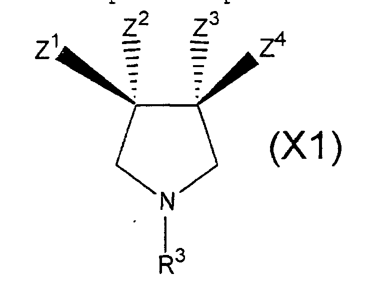 [wherein each of Z¹, Z², Z³ and Z⁴ independently means a hydrogen atom, a halogen atom, a hydroxyl group or a group represented by formula -O-R¹⁰ (wherein R¹⁰ means C₁₋₆ alkyl groups which may have 1 to 3 substituents selected from a substituent group A consisting of a halogen atom, a hydroxyl group, a methoxy group, a phenyl group, C₃₋₈ cycloalkyl groups and C₁₋₆ alkoxy groups, or C₃₋₈ cycloalkyl groups which may have 1 to 3 substituents selected from the substituent group A) ; any one of Z¹ and Z² as well as any one of Z³ and Z⁴ mean hydrogen atom; and R³ means benzyl group which may have 1 to 3 methoxy group(s) or nitro group (s) as substituent (s), or a hydrogen atom; with the proviso that a case (1) where a group which is not a hydrogen atom in Z¹ and Z² is the same with a group which is not a hydrogen atom in Z³ and Z⁴, and a case (2) where a group which is not a hydrogen atom in Z¹ and Z² is hydroxyl group, and a group which is not hydrogen atom in Z³ and Z⁴ is an ethoxy group or a chlorine atom are excluded]; or a salt thereof or a hydrate thereof.

3. The compound according to claim 2, wherein each of Z¹ and Z⁴ is a hydrogen atom; or a salt thereof or a hydrate thereof.

4. The compound according to claim 2, wherein each of Z¹ and Z³ is a hydrogen atom; or a salt thereof or a hydrate thereof.

5. The compound according to claim 2, wherein each of Z² and Z⁴ is a hydrogen atom; or a salt thereof or a hydrate thereof.

6. The compound according to claim 1, wherein R¹ is a group represented by formula -OR¹⁰ (wherein R¹⁰ has the same meaning as that of R¹⁰ defined in claim 1) ; or a salt thereof or a hydrate thereof.

7. The compound according to any one of claims 2 through 5, wherein at least one of Z¹ or Z² is a group represented by formula -OR¹⁰ (wherein R¹⁰ has the same meaning as that of R¹⁰ defined in claim 1; or a salt thereof or a hydrate thereof.

8. The compound according to any one of claims 1 through 7, wherein R³ is a hydrogen atom or a benzyl group; or a salt thereof or a hydrate thereof.

9. The compound according to any one of claims 1 through 7,
wherein R³ is a hydrogen atom; or a salt thereof or a hydrate thereof.

10. The compound according to any one of claims 1 through 9, wherein R¹⁰ is a hydroxyl group, a methoxy group, or a benzyloxy group; or a salt thereof or a hydrate thereof.

11. The compound according to claim 1 or 2, wherein said compound is selected from the group consisting of
(3R,4R)-3-hydroxy-4-methoxypyrrolidine,
(3S,4S)-3-hydroxy-4-methoxypyrrolidine,
(3R,4S)-3-hydroxy-4-methoxypyrrolidine, and
(3S,4R)-3-hydroxy-4-methoxypyrrolidine; or a salt thereof or a hydrate thereof.

12. The compound according to any one of claims 1 through 11,
wherein said salt is hydrochloride or oxalate; or a salt thereof or a hydrate thereof.

13. A process for producing 3-hydroxy-4-methoxypyrrolidine (6y) represented by the following formula (6y): 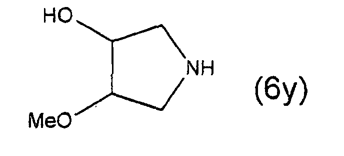 comprising the steps of:
subjecting a compound (1y) represented by formula (1y) to oxidation reaction to obtain a compound (2y) represented by formula (2y); 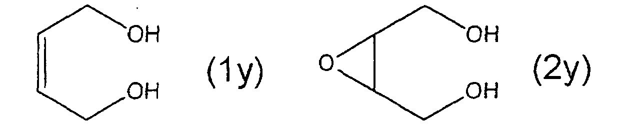 then, converting two hydroxyl groups in said compound (2y) into leaving groups to obtain a compound (3y) represented by formula (3y) (wherein L¹ means a halogen atom, a methylsulfonyloxy group, a p-toluenesulfonyloxy group, or a methanesulfonyloxy group); 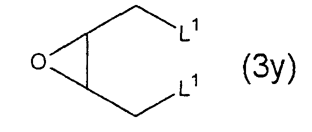 then, allowing said compound (3y) to react with an amine derivative represented by general formula R^{3a}-NH₂ (wherein R^{3a} means a benzyl group which may have 1 to 3 methoxy group(s) or nitro group(s) as substituent(s)) to obtain a compound (4y) represented by formula (4y) (wherein R^{3a} has the same meaning as defined above); 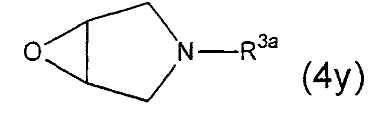 then, allowing said compound (4y) to react with alkaline metal methoxide salt to obtain a compound (5y) represented by formula (5y) (wherein R^{3a} has the same meaning as defined above); and 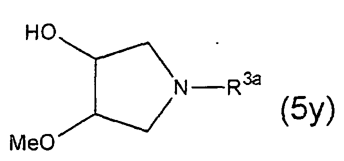 then, releasing R^{3a} in said compound (5y); or a salt thereof or a hydrate thereof.

14. A process for producing 3-hydroxy-4-methoxypyrrolidine (6y) represented by the following formula (6y): 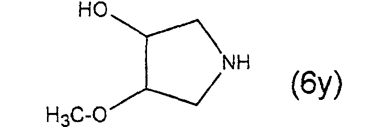 comprising the steps of:
allowing a compound (4y) represented by formula (4y) (wherein R^{3a} means a benzyl group which may have 1 to 3 methoxy group(s) or nitro group (s) as substituent (s)) to react with alkaline metal methoxide salt to obtain a compound (5y) represented by formula (5y) (wherein R^{3a} has the same meaning as defined above); 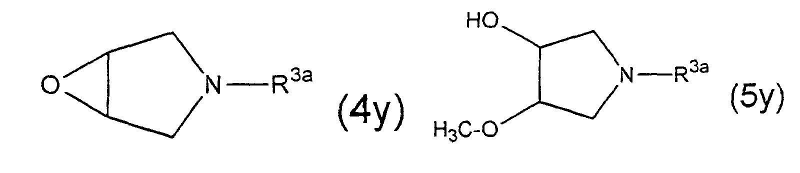 and then, releasing R^{3a} in said compound (5y); or a salt thereof or a hydrate thereof.

15. A process for producing 3-hydroxy-4-methoxypyrrolidine (8z) represented by formula (8z): 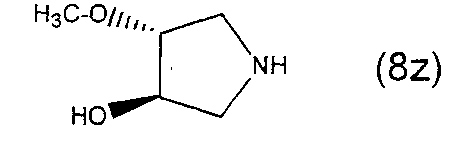 comprising the steps of:
allowing a compound (1z) represented by the following formula (1z) (wherein R⁵ means C₁₋₆ alkyl groups, a benzyl group or a hydrogen atom) to react with a compound represented by formula Ar-CHO (wherein Ar means a phenyl group which may have 1 to 3 substituent(s) selected from the group consisting of a nitro group and a methoxy group) to obtain a compound (2z) represented by formula (2z) (wherein each of Ar and R⁵ has the same meaning as defined above); 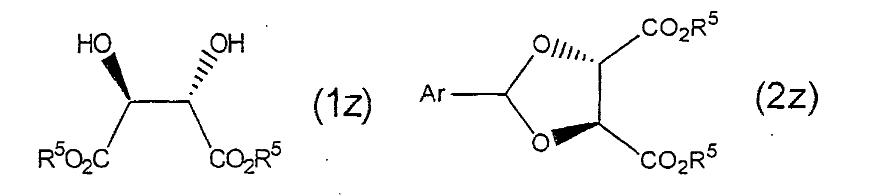 then, subjecting said compound (2z) to reductive reaction to obtain a compound (3z) represented by formula (3z) (wherein Ar has the same meaning as defined above); 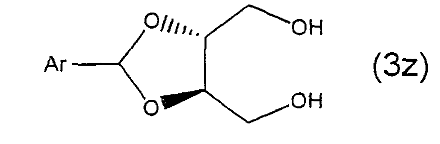 then, converting hydroxyl groups in said compound (3z) into leaving groups to obtain a compound (4z) represented by formula (4z) (wherein Ar has the same meaning as defined above, and L¹ means a halogen atom, a methanesulfonyloxy group, a p-toluenesulfonyl group or a trifluoromethylsulfonyloxy group); 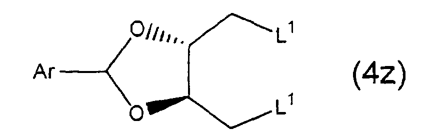 then, subjecting said compound (4z) to reductive reaction to obtain a compound (5z) represented by formula (5z) (wherein each of Ar and L¹ has the same meaning as defined above); 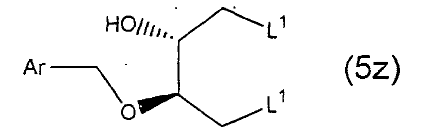 then, allowing said compound (5z) to react with a compound represented by formula R^{3a} -NH₂ (wherein R^{3a} means a benzyl group which may have 1 to 3 methoxy group(s) or nitro group(s) as substituent (s)) to obtain a compound (6z) represented by formula (6z) (wherein each of Ar and R^{3a} has the same meaning as defined above); 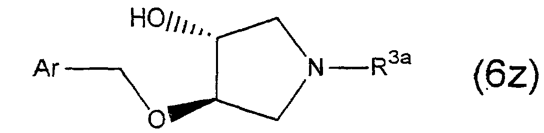 then, subjecting said compound (6z) to methylating reaction to obtain a compound (7z) represented by formula (7z) (wherein each of Ar and R^{3a} has the same meaning as defined above); and 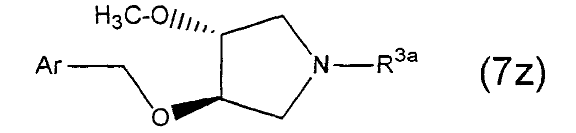 then, subjecting protecting group in said compound (7z) to deprotection; or a salt thereof or a hydrate thereof.

16. A process for producing 3-hydroxy-4-methoxypyrrolidine (8z) represented by formula (8z): 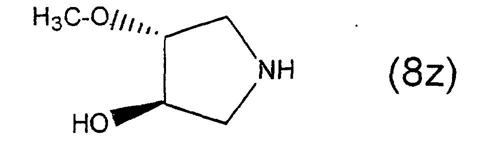 comprising the steps of:
subjecting a compound (4z) represented by formula (4z) (wherein Ar means a phenyl group which may have 1 to 3 substituent(s) selected from the group consisting of a ni tro group and a methoxy group, and L¹ means a halogen atom, a methanesulfonyloxy group, a p-toluenesulfonyl group or a trifluoromethylsulfonyloxy group) to reductive reaction to obtain a compound (5z) represented by formula (5z) (wherein each of Ar and L¹ has the same meaning as defined above); 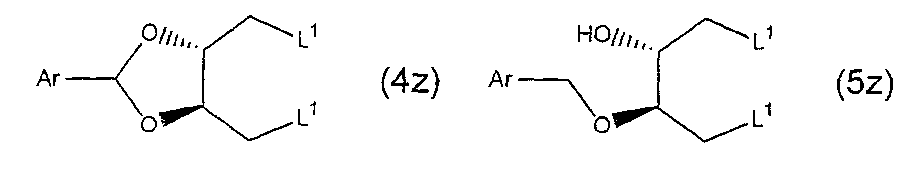 then, allowing said compound (5z) to react with formula R^{3a}-NH₂ (wherein R^{3a} means a benzyl group which may have 1 to 3 methoxy group(s) or nitro group(s) as substituent(s)) to obtain a compound (6z) represented by formula (6z) (wherein each of Ar and R^{3a} has the same meaning as defined above); 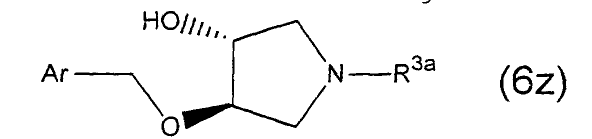 then, subjecting said compound (6z) to methylating reaction to obtain a compound (7z) represented by formula (7z) (wherein each of Ar and R^{3a} has the same meaning as defined above); and 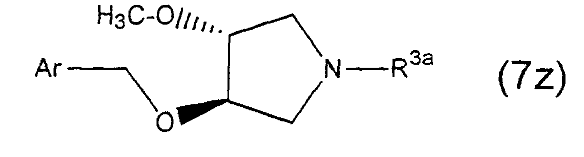 then, subjecting protecting group in said compound (7z) to deprotection; or a salt thereof, or a hydrate thereof.
